Europäisches Patentamt

European Patent Office

Office européen des brevets

(11) Veröffentlichungsnummer: **0 442 131 A1**

# EUROPÄISCHE PATENTANMELDUNG

(21) Anmeldenummer: 90125259.3

(22) Anmeldetag: **21.12.90**

(51) Int. Cl.5: **C07C 43/23**, C07C 41/16, C08G 18/32

(30) Priorität: **14.02.90 DE 4004493**

(43) Veröffentlichungstag der Anmeldung:
**21.08.91 Patentblatt 91/34**

(84) Benannte Vertragsstaaten:
**CH DE FR GB IT LI**

(71) Anmelder: **BAYER AG**

**W-5090 Leverkusen 1 Bayerwerk(DE)**

(72) Erfinder: **Lorenz, Otto, Dr., Fachhochschule Aachen**
**Kurbrunnenstrasse 22**
**W-5100 Aachen(DE)**

(54) **4,4'-Di-(Omega-hydroxyalkoxy)biphenyl, Verfahren zu dessen Herstellung und dessen Verwendung zur Herstellung von Polyurethanen.**

(57) Die vorliegende Erfindung betrifft die neuen Verbindungen 4,4'-Di-($\omega$-hydroxyalkoxy)biphenyl (alkoxy = $C_8$-$C_{13}$), insbesondere 4,4'-Di-(11-hydroxyundecanoxy)biphenyl (I) sowie deren Herstellung und Verwendung zur Herstellung segmentierter Polyurethane.

EP 0 442 131 A1

Die vorliegende Erfindung betrifft die neuen Verbindungen 4,4'-Di-(ω-hydroxyalkoxy)biphenyl (alkoxy = $C_8$-$C_{13}$), insbesondere 4,4'-Di-(11-hydroxyundecanoxy) biphenyl (I) sowie deren Herstellung und Verwendung zur Herstellung segmentierter Polyurethane.

Derartige Verbindungen sind bisher nicht bekannt geworden. Diese Verbindungen können nach analogen, an sich bekannten Verfahren z.B. aus 4,4'-Dihydroxybiphenyl und ω-Halogenalkanolen (Halogen = Cl, Br) hergestellt werden (vgl. z.B. B. Riek et al. Makromol. Chem. Rapid. Comm. 6 (1985), 291).

Es wurde überraschend gefunden, daß diese Verbindungen, insbesondere I wertvolle Komponenten zum Aufbau von segmentierten Polyurethanen sind. Damit erhaltene Polyurethane, die in an sich bekannter Weise hergestellt werden (vgl. z.B. D. Dieterich in Houben-Weyl: Methoden der Organischen Chemie Band E 20, S. 1568 - 1571, 1629 -1641; G. Thieme Verlag Stuttgart 1987), unterscheiden sich vorteilhaft von analog aufgebauten Polyurethanen, die z.B, Butandiol (1.4) oder bekannte 4,4'-Di-(ω-hydroxyalkoxy)-biphenyle enthalten, wie z.B. die entspechende Hexan- oder Propanverbindung.

Die Verwendung der neuen Verbindungen, insbesondere von I beim Aufbau von segmentierten Polyurethanen, z.B. anstelle des vielfach eingesetzten Butandiol (1,4) hat ein besonders günstiges Eigenschaftsbild der so erhaltenen Produkte zur Folge.

Insbesondere werden hohe Spannungswerte beobachtet, sowie thermische Übergänge, die auf das Vorliegen ausgeprägter Mesophasen hindeuten.

Es scheint, daß infolge der großen Spacerlänge eine vorteilhafte Entkopplung von mesogenem Bereich und Urethanteil eintritt.

Eine zusätzliche chemische Vernetzung unterhalb der thermischen Übergänge führt zu einer weiteren Verbesserung der mechanischen Dehneigenschaften. Als Vernetzungsmittel eignen sich beispielsweise Melaminharze.

Der Gegenstand der Erfindung soll anhand der folgenden Beispiele noch näher erläutert werden.

Beispiel 1

Herstellung von 4,4-Di(11-hydroxyundecanoxy)biphenyl (I)
Ausgangsstoffe:  4,4-Dihydroxybiphenyl (DHB)
HO-$C_6H_4$-$C_6H_4$-OH
KOH (Plätzchen)
11-Bromundecanol Br-$(CH_2)_{11}$-OH
Lösungsmittel:  Ethanol
Geräte:  500 ml Dreihalsrundkolben, Rührer, Kontaktthermometer, Pilzheizhaube, Rückflußkühler.

0,054 Mol (10 g) DHB und 0,12 Mol (6,8 g) KOH werden in 200 ml Ethanol gelöst.

Dann werden 0,12 Mol (30,15 g) 11-Bromundecanol zugegeben und 16 h unter Rückfluß erhitzt.

Anschließend wird die Lösung in 1 l dest. Wasser eingegossen.

Der Niederschlag wird abgenutscht und mit 50 ml 0,1 n NaOH gewaschen. Danach wird mit dest. Wasser neutral gewaschen.

Nach dem Trocknen wird aus einem Gemisch von Ethanol mit Tetrahydrofuran im Volumenverhältnis 5/3 zweimal umkristallisiert.

Ausbeute: ca. 50 % Fp. 159° C.

Beispiele 4-5 und Vergleichsbeispiele 2-3.

Synthese segmentierter Polyurethane

In das entwässerte Makrodiol rührte man bei ca. 80° C die DO-Komponente ein, die sich im Makrodiol nicht vollständig löste. Das DI wurde ebenfalls bei 80° C unter Rühren eingetropft, danach heizte man die Mischung langsam auf 110° C auf. Bei Verwendung von IPDI oder Gemischen aus IPDI und HDI (mit IPDI als Hauptkomponente) bildete sich bei Verwendung von 3-DO innerhalb einer Stunde eine klare Lösung. Bei Verwendung von 6-DO bzw. 11-DO war ein Zusatz von N-Methylpyrrolidon (NMP) (10-20 % bezogen auf die Gesamtmasse) erforderlich, um eine klare Lösung zu bilden. Setzte man als DI ausschließlich HDI ein, so entstanden in allen Fällen nur nach Zugabe von NMP klare Lösungen. Hinsichtlich der Bildung nichttrüber Lösungen erwies sich PCL günstiger als PTMO. Der NCO-Umsatz wurde titrimetrisch verfolgt. Die Reaktionszeit für die Bildung des NCO-Prepolymers betrug im allgemeinen 3 h bei 110° C. Nach Abkühlen auf ca. 80° C wurde das NCO-Prepolymer in wasserfreiem THF gelöst, so daß der Feststoffgehalt 30 bis 35° C betrug.

Bei der Kettenverlängerung mit 1,3-Diaminopropan bzw. N-Methyl-1,3-diaminopropan wurde die Lösung des NCO-Prepolymers (Lösemittel: THF bzw. Gemisch aus THF und NMP) in eine 2,5proz. Lösung des Diamins in THF eingerührt.

2

Nichtionische Polyurethane, Rezepturen, DSC-Ereignisse und mechanische Eigenschaften

| Edukte | 2 | 3 | 4 | 5 |
|---|---|---|---|---|
| PTMO 2000[b]/mol | - | - | - | - |
| PCL 1250[a]/mol | 0,8 | 0,8 | 0,8 | 0,6 |
| BD[a]/mol | 0,6 | - | - | - |
| 6-DO[a]/mol | - | 0,6 | - | - |
| 11-DO[a]/mol~ I | - | - | 0,6 | 0,6 |
| HDI[a]/mol | 0,4 | 0,4 | 0,4 | 0,4 |
| 1,3-DAP[b]/mol | 1,6 | 1,6 | 1,6 | 1,6 |
| N-Me-1,3-DAP[b]/mol | - | - | - | 1,2 |
| $T_g$/K | 231 | 231 | 226 | 226 |
| $T^c$/K (Aufheizen) | (320) | (320) | (326) | (326) |
| | - | - | 357 | 354 |
| | - | - | 396 | 398 |
| | | | (417) | |
| $T^d$/K (Abkühlen) | | | 339 | 337 |
| $\sigma_{50}{}^d$/Ncm$^{-2}$ (20° C) | 95 | 135 | 345 | 335 |
| (40° C) | 75 | 70 | 270 | 195 |
| (60° C) | 55 | 30 | 130 | 65 |
| (80° C) | n.b. | n.b. | n.b. | n.b |

a HDI = 1,6-Hexamethylendiisocyanat; BD = 1,4-Butandiol; 3-DO, 6-DO, 11-DO = 4,4-Di($\omega$-hydroxyalkoxy)-bisphenyl mit n = 3,6 bzw. 11 Methylengruppen, PCL = Poly(caprolacton), $\overline{M}_n$ = 1250 g Mol$^{-1}$; IPDI = 3-Isocyanatomethyl-3,5,5-trimethylcyclohexylisocyanat.
b PTMO 2000 = Polyl(tetramethylenoxid), $\overline{M}_n$ = 2000 g mol$^{-1}$; 1,3-DAP = 1,3-Diaminopropan; N-Me-1,3-DAP = N-Methyl-1,3-diaminopropan.
c Thermische Ereignisse des zweiten Aufheiz- und Abkühlvorganges.
d Spannungswerte bei 50 % Dehnung.

**Patentansprüche**

1. 4,4'-Di($\omega$-hydroxyalkoxy)biphenyl (alkoxy = $C_8$-$C_{13}$).

2. 4,4'-Di(11-hydroxyundecanoxy)biphenyl (I).

3. Verfahren zur Herstellung von Verbindungen gemäß Anspruch 1 oder 2, dadurch gekennzeichnet, daß man 4,4'-Dihydroxybiphenyl und ein Halogenalkanol mit 8-13 C-Atomen (Halogen = Br, Cl) umsetzt.

4. Verwendung von Verbindungen gemäß Anspruch 1 oder 2 zur Herstellung von segmentierten Polyurethanen.

3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | BE-A-6 969 84 (PFIZER CORP.) <br> * S. 8-9, Beispiele 1-3; Anspruch 1 * <br> — — — | 1 | C 07 C 43/23 <br> C 07 C 41/16 <br> C 08 G 18/32 |
| A | GB-A-1 019 566 (ALLIED CHEMICAL CORP.) <br> * Seite 2, Zeile 6 - Seite 2, Zeile 32 * <br> — — — | 1,4 | |
| A | US-A-2 789 965 (D.D. REYNOLDS ET AL.) <br> * Spalte 5, Zeile 8 - Spalte 5, Zeile 22 * <br> — — — | 1 | |
| A | US-A-2 140 824 (C.C. VERNON) <br> * Seite 1, Spalte 1, Zeile 45 - Seite 1, Spalte 2, Zeile 48 * <br> — — — | 1,3 | |
| A | PATENT ABSTRACTS OF JAPAN vol. 10, no. 235 <br> (C-366)(2291) 14 August 1986, <br> & JP-A-61 68436 (CHISSO CORP.) 08 April 1986, <br> * das ganze Dokument * <br> — — — | 1,3 | |
| A | US-A-4 170 711 (C.M. ORLANDO ET AL.) <br> * Spalte 5, Zeile 11 - Spalte 5, Zeile 39 * <br> — — — | 1 | |
| D,A | Die Makromolekulare Chemie Rapid Communications <br> & Wepf Verlag, Basel, CH Seiten 291 - 299; B. Reck et al.: <br> "Combined liquid crystalline polymers: Mesogens in the <br> main chain and as side groups" <br> * Seite 297, Absatz 7 * <br> — — — — — | 1 | **RECHERCHIERTE SACHGEBIETE (Int. Cl.5)** <br><br> C 07 C <br> C 08 G |

Der vorliegende Recherchenbericht wurde für alle Patentansprüche erstellt

| Recherchenort | Abschlußdatum der Recherche | Prüfer |
|---|---|---|
| Berlin | 24 Mai 91 | PROBERT C.L. |